# EUROPEAN PATENT APPLICATION

(11) **EP 1 566 145 A1**
(43) Date of publication of application: **24.08.2005**
(21) Application number: 05003625.0
(22) Date of filing: 19.02.2005
(51) Int. Cl.: A61B 5/15

(54) **Improved plug for test tubes for taking blood samples**

(30) Priority: 20.02.2004 IT PD20040048
(71) Applicant: VIZAPLASTIK s.r.l., 36070 San Pietro Mussolino VI (IT)
(72) Inventor: Vallarsa, Stefano, 36070 San Pietro Mussolino VI (IT); Rossi, Radames, 35010 Vigonza PD (IT)

(57) **Abstract**

Plug for test tubes mainly for taking blood samples consisting of the plug itself (11) made of elastomer with upper stop flange (8) to close the test tube (12, 13, 14) and a cover (1) made of compact plastic material which covers the upper flange (8) of the plug, coupling irreversibly, and partially covers the outer upper section of the test tube (12, 13, 14) characterised in that the cover (1) possesses at least two lugs (7) protruding on the inner surface which engage on the lower edge (10) of the flange (8) of the elastomer plug (11) and is provided on the lower surface of the head (2) with an annular descending pronounced sturdy appendix (6) which necessarily engages on the conchoidal configuration (9) of the upper part of the elastomer plug (11) thus providing a sealed chamber since the head surface (2) above of the cover (11) is without apertures.

## Description

It is known that a large number of types of "plugs" exist on the market for test tubes for taking blood samples or for handling of biological liquids.

Taking account of the purpose they are required to fulfil associated with practicality and safety of use in addition to limitation of costs, the various products of the various manufacturers constitute a wide-ranging field of patents.

The so-called "plugs" for test tubes for taking blood samples or for handling biological liquids consist of the plug itself made of elastomer provided with an upper stop flange which is inserted into the test tube to close it and a cover made of compact plastic material which covers and irreversibly couples with the elastomer plug and covers a portion of the upper part of the test tube creating with said part a space.

The difficulty to be overcome in obtaining the above-mentioned plugs lies above all in the production of covers which must be configured inside so that they can couple almost integrally with the elastomer plug they cover, do not adhere to the part of the test tube they cover, do not exceed a certain external diameter with respect to the test tubes, especially for Φ=16 test tubes, which would make them unusable due to the impossibility of inserting the closed assembled test tube (with the elastomer plug) in corresponding holders provided in blood sampling equipment or adapters for analysis machines, such as centrifuges.

One of the drawbacks of the "plugs" existing on the market is due to the fact that the cover in the central part of the head is open so that the upper surface of the elastomer plug, even if previously sterilised, in practice does not continue to be protected.

When the needle of the syringe is inserted through the elastomer plug, any contaminating elements that may be deposited on the surface of the conchoidal cavity can enter the inside (sterile) of the test tube, and can therefore distort subsequent analyses (of the blood).

To remedy said problem, some manufacturers apply a further cover on the head of the cover.

This does not solve the problem because the wall of said additional cover cannot be penetrated by the needle of the syringe and therefore said cover is previously removed.

A further problem of the manufacturers is to provide, between the lower surface of the cover head upper wall and the elastomer plug, spaces in which to confine any drops of blood dispersed during insertion and removal of the needle of the syringe through the elastomer plug in order to prevent possible contact between the operators and said particles of blood.

The solutions adopted existing on the market are not exhaustive.

The aim of the present patent is to produce a blood sample test tube plug able to overcome, in a simple, inexpensive, practical and safe way, all the main drawbacks and construction difficulties of the blood sample test tube plugs existing on the market, making an important contribution in terms of innovative details.

The above aim is achieved by a plug with one or more of the characteristics described below:
- the blood sample test tube plug comprises only two elements, i.e.:
   o the elastomer plug itself provided with upper stop flange to be inserted in the test tube to be closed with the advantage if necessary of a conchoidal recess in the upper central area;
   o the compact plastic cover able to cover the elastomer plug, coupling in a practically irreversible manner and acting on the upper stop flange, without coming into contact with the test tube of which it covers an upper section and having such a fine perimeter wall as to exceed as little as possible the diameter of the test tube;
- the cover, basically cylindrical, is obtained inexpensively in compact plastic material (preferably polyethylene) by simple injection into the cavity of the mould consisting of punch and die with normal extraction of the punch at the end of the injection process characterised in that:
   o the cylindrical perimeter wall is fine in order to minimise the difference in diameter with respect to the diameter of the test tube but such that its internal diameter is larger than the external diameter of the test tube, thus creating a space between said internal diameter of the cover and said external diameter of the test tube;
   o the head wall is continuous without central apertures, preventing direct communication of the conchoidal surface on the top of the seal flange of the elastomer plug with the outside once it has been inserted;
   o the internal surface of the head has a pronounced and sturdy annular appendix directed towards the inside coaxial with the lateral cylindrical wall and having a diameter smaller than the maximum aperture of the conchoidal cavity on the top of the stop flange of the elastomer plug;
   o the internal surface of the cylindrical wall has at least two lugs protruding step-like, the distance of which from the lower edge of the annular appendix directed towards the inside present on the inner surface of the head is such that once the elastomer plug has been inserted, the elastomer plug is engaged simultaneously at the bottom in its stop flange and at the top in the conchoidal surface, providing a practically irreversible forced coupling;
   o the outer cylindrical surface, excluding a portion towards the head, is provided with narrow spaced ribs arranged longitudinally for improved grip;
   o the portion of head wall bounded by the annular appendix present on the inner surface is very fine so that it can be easily perforated by the needle of a syringe;
   o the continuous head wall without central apertures, preventing direct communication of the chamber defined between it and the conchoidal surface on the top of the seal flange of the elastomer plug with the outside once said plug has been inserted, said wall being made of polyethylene, which is not airtight, permits stabilisation of the atmospheric pressure inside the above-mentioned chamber.

A particular characteristic is that the space defined by the inner annular appendix of the head wall, by the surface of the conchoidal cavity of the flange of the elastomer plug bound by the above-mentioned appendix and by the above film of the head wall, continues to be sterile after sterilisation treatment of the assembly, since it does not communicate with the outside, and consequently the surface of the conchoidal cavity of the flange which is perforated by the needle of the syringe continues to be sterile.

Another peculiarity is the fact that in addition to the guaranteed sterility of the surface of the conchoidal cavity, the above-mentioned space provides a guarantee against the dispersion of any drops of blood left when the needle of the syringe is extracted.

The above description is clarified by examination of the attached drawings of a preferred non-limiting embodiment.

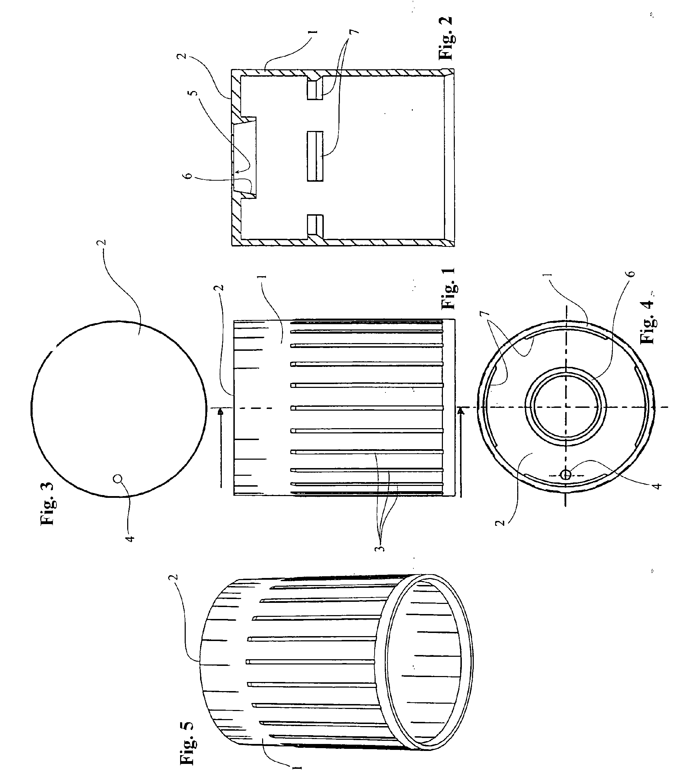

Fig. 1 is a side view of the cover.
Fig. 2 is a section according to the median plane A-A.
Fig. 3 shows the cover seen from above.
Fig. 4 shows the cover seen from below.
Fig. 5 is a perspective view of the cover taken partially from below.

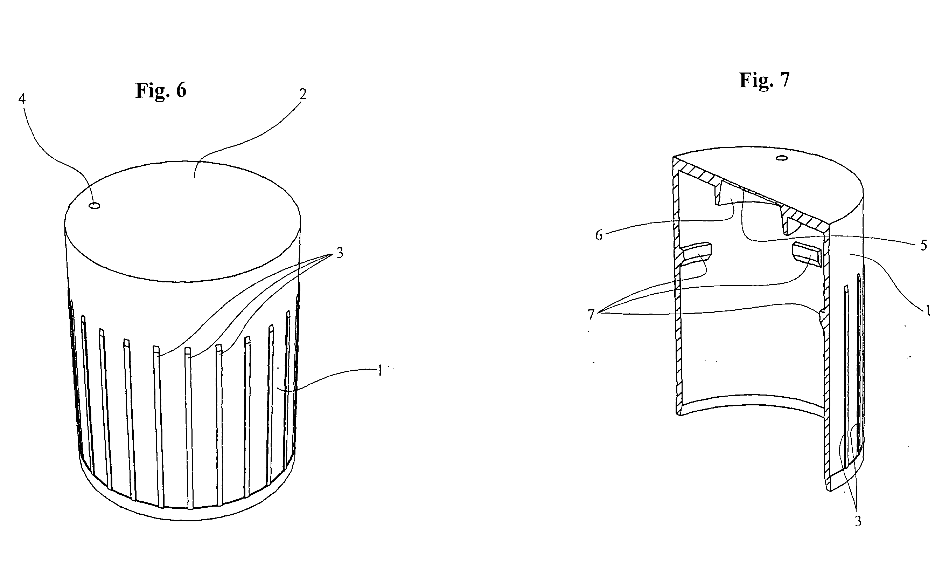

Fig. 6 is a perspective view of the cover taken partially from above.
Fig. 7 is a section view of the cover corresponding to Fig. 6.

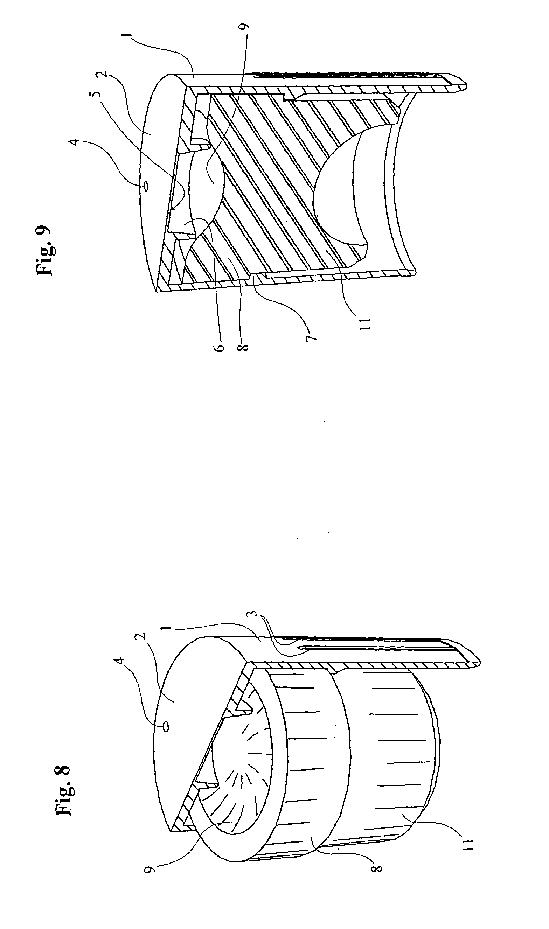

Fig. 8 shows the cover sectioned with the elastomer plug inserted.
Fig. 9 shows the cover and elastomer plug inserted, both sectioned.

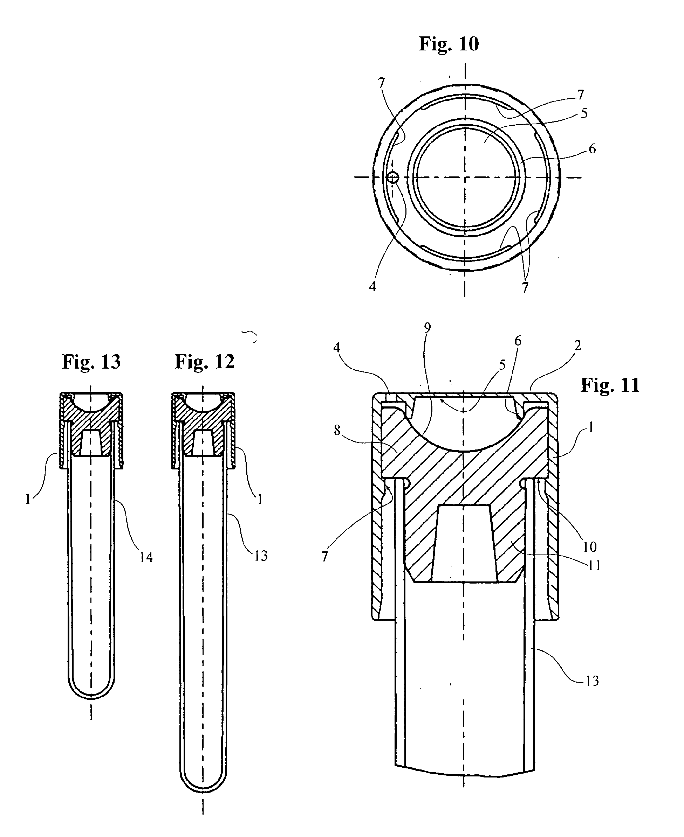

Fig. 10 is a view of the cover shown from below.
Fig. 11 shows, in an enlarged section, the cover, the elastomer plug and part of the test tube assembled together.
Fig. 12 and 13 are representations corresponding to Fig. 11 with whole test tubes of two different lengths.

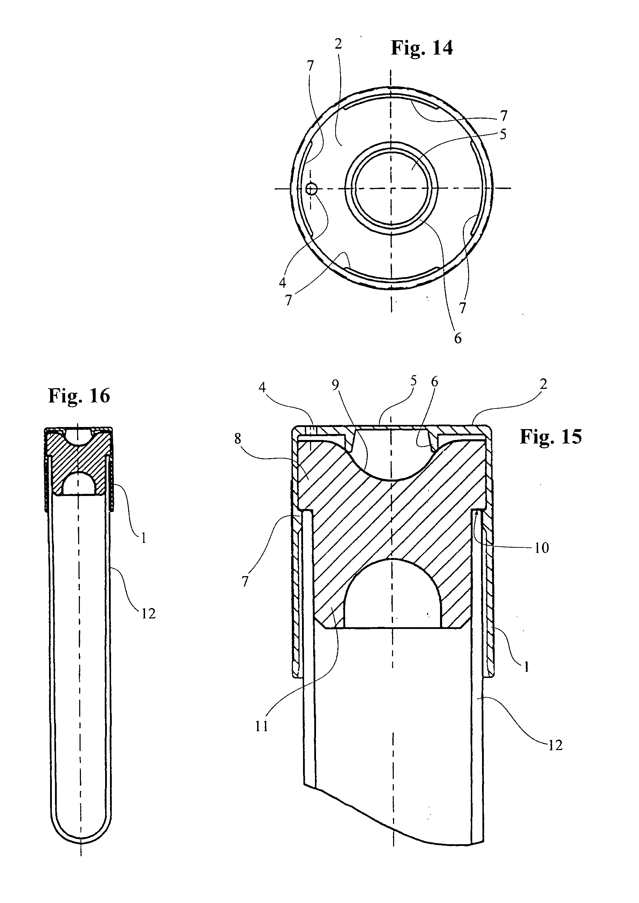

Fig. 14 is a view from below of the cover corresponding to Fig. 10.

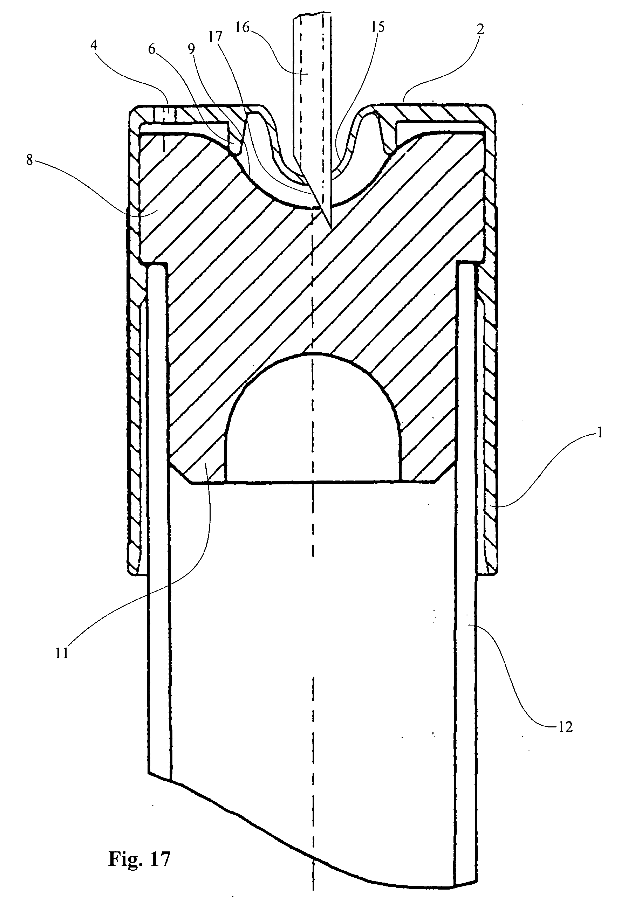

Fig. 15 shows, in an enlarged section, the cover, the elastomer plug and part of the test tube assembled together, with the difference compared to Fig. 11 that in the case of a test tube with larger diameter (16 mm) the space between the cylindrical wall of the cover and the test tube is reduced to a minimum.
Fig. 16 is a representation corresponding to Fig. 14 with whole test tube.
Fig. 17 shows, in an enlarged section, the cover with the top wall with conchoidal recess in the central part near the conchoidal surface of the elastomer plug and the needle in the initial phase of perforation of the cover wall and elastomer plug assembly.

From examination of the figures previously referred to it can be seen that the test tube plug for taking blood samples and handling biological liquids comprises both the elastomer plug 8, 11 itself with cylindrical configuration, the lower diameter of which is designed to be inserted in the test tube 12, 13, 14, and the cover 1 which has on its inner lateral surface four step-like protrusions 7 and which covers the upper widened part configured as a flange 8 of the above-mentioned elastomer plug 11 presenting said flange 8, in the lower part a crown 10 with undercut surface, and in the upper part in the central area a conchoidal cavity.

According to the patent, the cover 1 as previously said has on its cylindrical inner surface four protrusions 7, equally spaced from each other, and a head wall 2 without apertures provided on the lower surface with a pronounced and sturdy annular appendix 6 coaxial with the cylindrical configuration of the cover 1 directed towards the inside.

A particular characteristic of the invention is the fact that with completion of insertion of the elastomer plug 11 inside the cover 1, with the flange 8 with lower stop 10 an almost irreversible coupling is provided between the protrusions 7 and the stop crown 10 and the lower edge of the annular appendix 6 against the surface of the conchoidal cavity 9 on the top of the flange 8.

The peculiarity of the type of coupling between the cover 1 made of compact plastic and the elastomer plug 11 of the present patent also constitutes a non-negligible innovation as the surface of the conchoidal cavity 9 which is perforated by the needle of the syringe is protected as regards maintenance of sterilisation efficiency.

In fact the space above determined by the annular appendix 6 and the inner surface 5 of the head wall 2 of the cover 1, since it is a closed space not communicating with the outside, protects the surface of the conchoidal cavity 9 against external agents.

A further advantage of said closed space is that it constitutes a protection against the dispersion of any drops of blood left by the needle of the syringe during extraction.

A further advantage derives from the fact that since the diameter of the needle inlet hole substantially coincides with the diameter of the needle after extraction of said needle from the elastomer plug 11, its already partly cleaned surface is scraped to remove any traces of blood left by its tip and deposited on the outer surface.

It is important to observe that prior to perforation of the head wall 2 of the cover 1 the upper surface of said head is a smooth, flat continuous surface.

The characteristics of said outer upper surface of the head 2 permit rapid, safe and effective cleaning of the surface itself before insertion of the needle in the plug, thus preventing the needle coming into contact with an unclean surface, becoming contaminated and introducing undesired elements into the test tube. Furthermore the above-mentioned characteristics of said surface 2 ensure perfect seal of the chamber below in combination with the plug, thus maintaining the sterility of the chamber obtained by assembly of the cover 1 with the plug.

Although from examination of the drawings from 1 to 16 said smooth continuous surface 5, 15 is flat, advantageously said surface as represented in Fig. 17 features a conchoidal recess in the central part. Said recess which involves the related wall in the central area of the cover, when the elastomer plug is inserted in said cover, is so close to the conchoidal surface of the plug that during perforation the front slanting face of the needle is still engaged in perforation of the cover wall when the tip has already begun penetrating the conchoidal surface of the elastomer plug below. The effect of resistance to the penetration transmitted to the needle is felt by the patient (in the case of direct blood sampling) as if the needle were crossing the elastomer plug only.

During the initial perforation phase the front part 17 with slanting face of the needle 16 is still engaged in perforation of the wall 15 of the cover head wall 2 when the tip of said slanting face already begins to enter the conchoidal surface 9 of the elastomer plug 11.

To facilitate gripping of the cover 1, it is provided on the outer surface with small ribs 3 arranged longitudinally and appropriately spaced which do not reach the top, leaving the upper strip adjacent to the head free in order not to negatively affect, once injection into the mould has been completed, the pliability of said strip necessary for extraction of the punch which must pass over the protrusions 7 present inside which, instead of behaving like undercuts, can move towards the outside.

A further advantage derives from provision on the upper surface of the cover head wall 2 of at least one hole 4 which, during injection moulding of the cover, permits extraction of the male punch and, during assembly of the cover 1 with the plug, permits outflow of the air between said cover and said plug. Positioning of said hole 4 on the peripheral part of the upper surface of the head wall preserves the seal of the drip-catcher chamber between the cover 1 and the plug 11.

Any embodiment, also for the purposes of improvement, that a person skilled in the art may implement following the precepts of the present patent remains within the scope of said patent.

## Claims

1. Improved plug for test tubes for taking blood samples, **characterised in that** it comprises: the plug itself (11) made of elastomer, the lower part of which inserted in the test tube (12, 13, 14) closes and seals it, and the upper part of which (8) necessarily couples with the cavity of the cover (1); a cover (1) which covers the upper part (8) of the elastomer plug, said cover (1) having head wall (2) with continuous outer surface.

2. Improved plug for test tubes for taking blood samples, as claimed in claim 1 **characterised in that** said head wall (2) of the cover (1) is flat.

3. Improved plug for test tubes for taking blood samples, as claimed in claim 1 **characterised in that** said head wall (2) of the cover (1) is smooth.

4. Improved plug for test tubes for taking blood samples, as claimed in claim 1 **characterised in that** the plug (11) has at the top (8) a cylindrical configuration complementary to the cover (1) by which it is retained, said cover (1) being configured so as to provide in combination with said plug (11) a sealed closed chamber consisting of the head wall (2) of the cover (1), a side strip of the cylindrical surface of the cover (1) and the upper surface (8) of the plug (11).

5. Improved plug for test tubes for taking blood samples, as claimed in claim 1 **characterised in that** said closed chamber is alternatively created laterally by a continuous circular appendix (6) integral with the head wall (2) of the cover (1) or integral below the plug (11), leaving in the cover (1) a concentric circular crown space outside said closed chamber.

6. Improved plug for test tubes for taking blood samples, as claimed in claim 1 or 4 or 5 **characterised in that** said head wall (2) of the cover (1) is thinner in the centre (5) in order to constitute a barrier that can be easily perforated by any needle when inserted through the plug.

7. Improved plug for test tubes for taking blood samples, as claimed in claim 1 **characterised in that** the upper surface of the head wall (2) of the cover (1) has at least one hole (4) which permits, during injection moulding of the cover (1), extraction of the male punch and, during assembly of the cover (1) and plug (11), outflow of the air from between said cover (1) and said plug (11).

8. Improved plug for test tubes for taking blood samples, as claimed in claim 7 and 4 or 5 **characterised in that** said at least one hole (4) is present on the outer part of the head wall (2) of the cover (1) therefore not affecting the seal of the drip-catcher chamber provided centrally between the cover (1) and the plug (11).

9. Improved plug for test tubes for taking blood samples, as claimed in claim 7 or 8 **characterised in that** said hole (4) is closed during assembly of the cover (1) with the plug (11) by part of said circular appendix (6) integral with the plug (11).

10. Improved plug for test tubes for taking blood samples, as claimed in one or more of the preceding claims **characterised in that** said basically cylindrical elastomer plug (11), the lower part of which (11) is inserted in the test tube (11, 13, 14) to close said test tube and the upper widened part of which, configured as a flange (8), having the function of coupling with the inner part of the cover (1) adjacent to the head, has at the level of the widened part an undercut perimeter surface (10) acting as a stop.

11. Improved plug for test tubes for taking blood samples, as claimed in one or more of the preceding claims **characterised in that** said plug (11) is provided at the top with a conchoidal cavity (9).

12. Improved plug for test tubes for taking blood samples, as claimed in one or more of the preceding claims **characterised in that** said cover (1), basically cylindrical, made of compact plastic material, with the function of coupling in an almost irreversible manner with the upper widened part in the shape of a flange (8) of the elastomer plug (11) and covering the upper part of the test tube (12, 13, 14), maintaining minimum space all around, said cover (1) has on its inner cylindrical wall at least two step-like protrusions (7) equally spaced from one another coupling and stopping on the undercut surface (10) of the flange (8) of the elastomer plug (11).

13. Improved plug for test tubes for taking blood samples, as claimed in the first claim **characterised in that** the cover (1) assembled with the flange (8) of the elastomer plug (11) provides, with the central area (5) of the head wall (2), with the annular appendix (6) of the head wall (2) and with the conchoidal cavity (9) of the flange (8) of the plug (11) a closed space not accessible from the outside by contaminating elements thus protecting said space after sterilisation treatment.

14. Improved plug for test tubes for taking blood samples, as claimed in claim 2 or 3 **characterised in that** the closed space defined by the wall (5) of the head (2) of the cover (1), by the tubular appendix (6) and by the surface below of the conchoidal cavity (9) of the flange (8) guarantees retention of any drops of blood left by the needle of the syringe during its extraction.

15. Improved plug for test tubes for taking blood samples, as claimed in one of the preceding claims **characterised in that** the cover (1) is produced with the thickness of the cylindrical wall in particular at the level of the strip adjacent to the head able to permit flexibility and expansion of the protrusions (7) provided on the inner surface so that during extraction of the male punch the above-mentioned protrusions (7) do not behave as undercuts, thus overcoming the technical problem which until now has prevented said embodiment.

16. Improved plug for test tubes for taking blood samples, as claimed in one or more of the preceding claims **characterised in that** the film of plastic material (5) of the head wall (2) of the cover (1) performs a scraping cleaning action on the outer surface of the needle during both insertion and extraction, leaving any traces of foreign bodies outside during insertion and retaining inside the closed chamber any traces of blood left after extraction of the needle from the plug.

17. Improved plug for test tubes for taking blood samples, as claimed in one or more of the preceding claims **characterised in that** the front slanting face (17) of the needle (16) during perforation is simultaneously engaged in passing through the head wall (15) of the plug (2) and in penetrating with the tip of said front slanting face (17) the conchoidal surface (9) of the elastomer plug so that the resistance perceived by the needle (16) (and therefore the patient in the case of direct blood sampling) feels as if it were one single perforation.

18. Improved plug for test tubes for taking blood samples, as claimed in one or more of the preceding claims **characterised in that** inside the chamber defined by the head wall (5, 15) and the conchoidal surface (9) of the elastomer plug (11) which, once sterilised remains sterile as it does not communicate with the outside, the internal pressure has the same values as the external pressure since a material which is permeable to air, such as polyethylene, is used for the head wall (5, 15).
